# NOUVEAU FASCICULE DE BREVET EUROPEEN

(11) **EP 0 972 522 B2**
(45) Date de publication et mention de la décision concernant l'opposition: **31.10.2007**
(45) Mention de la délivrance du brevet: 07.05.2003
(21) Numéro de dépôt: 99401720.0
(22) Date de dépôt: 08.07.1999
(51) Int. Cl.: A61K 38/34, A61K 8/60, A61P 29/00, A61K 36/03

(54) **Composition anti-inflammatoire**
Entzündungshemmende Zusammensetzung
Anti-inflammatory composition

(30) Priorité: 15.07.1998 FR 9809055
(43) Date de publication de la demande: 19.01.2000
(73) Titulaire: L'ORÉAL, 75008 Paris (FR)
(72) Inventeur: Mahe, Yann, 91300 Morsang-Sur-Orge (FR); Billoni, Nelly, 95750 Valmondois (FR); Breton, Lionel, 78000 Versailles (FR); Bui-Bertrand, Lien, 91600 Savigny-Sur-Orge (FR)
(74) Mandataire: Allab, Myriam

(56) Documents cités:
- WO-A-95/08564
- WO-A2-98/25584
- FR-A- 2 710 340
- FR-A- 2 733 421
- FR-A- 2 753 903
- US-A- 5 028 592
- Watanabe T. et al., Brain Research Bulletin, vol. 32, pp. 311-314,(1993)

## Description

La présente invention a pour objet une composition comprenant à titre de principe actif au moins l'association en une quantité efficace d'un dérivé de l'hormone stimulatrice des mélanocytes de type α (α-MSH) ou de tout équivalent biologique fonctionnel, et d'un extrait d'algue d'origine marine ledit extrait étant une solution d'oligosaccharides obtenue par dépolymérisation enzymatique de polysaccharides membranaires d'algue brune. L'invention concerne également l'utilisation d'une telle association dans une composition pour combattre les désordres faisant intervenir un processus inflammatoire et l'utilisation d'une telle association pour la préparation d'une composition cosmétique.

L'inflammation (ou processus inflammatoire) est un ensemble de réactions biologiques qu'on retrouve dans toute l'échelle animale. Chez l'homme, deux malades sur trois présentent un syndrome inflammatoire. L'inflammation peut être localisée. Elle peut se définir comme la première réponse à toute agression locale par une série de réactions non spécifiques déclenchées quelle que soit la cause initiale et se déroulant en trois étapes successives : vasculaire, cellulo-vasculaire et fibrose tissulaire.
Il existe une gradation symptomatique de l'inflammation qui va du sentiment d'inconfort cutané, des tiraillements, des démangeaisons au gonflement, à la douleur, à la rougeur et/ou à la chaleur. Ces symptômes sont généralement dus à l'infiltration des tissus blessés par un oedème et/ou à la vasodilatation des capillaires.

Les signes de l'inflammation peuvent aller jusqu'à la fièvre, un état de malaise général et/ou une augmentation de la concentration de certaines protéines de plasma sanguin.
C'est un phénomène qui implique entre autres, une série de réactions cellulaires locales et la libération de cytokines et autres médiateurs tels que la substance P, les prostaglandines, les leukotriènes, la bradykinine, l'histamine ou encore la sérotonine.
L'inflammation se manifeste par une modification du flux sanguin avec, au niveau du site agressé, une augmentation de la perméabilité vasculaire entraînant une fuite de protéines plasmatiques vers le fluide extracellulaire, ainsi qu'une extravasation de cellules sanguines, notamment des leucocytes neutrophiles et des macrophages vers le site inflammatoire.

Ces phénomènes sont en fait le résultat de l'action des médiateurs de l'inflammation.
Parmi les facteurs impliqués dans ces phénomènes inflammatoires, on peut citer les cytokines dont en particulier l'interleukine 1-α, l'interleukine 1-β, l'interleukine 6, les facteurs de nécrose tumorale α et β (TNF-α et -β), les chimiokines comme l'interleukine 8 ou le facteur chimiotactique et activateur des monocytes (MCAF), ou encore d'autres facteurs chimiotactiques responsables du recrutement des cellules lymphocytaires, monocytaires, de Langerhans ou basophiles au niveau du site inflammatoire, tels que les leukotriènes B-4, ou encore d'autres facteurs impliqués dans la cascade inflammatoire, tels que l'acide arachidonique, ou les prostaglandines, dont en particulier les prostaglandines E2.

Les phénomènes inflammatoires sont associés à de nombreux désordres allant du simple inconfort cutané jusqu'à des états pathologiques.
On peut citer à titre d'exemple les désordres cutanées telles que les peaux sensibles, l'inconfort cutané, les tiraillements cutanés, les démangeaisons cutanés, le gonflement cutané, la douleur cutanée, la rougeur cutanée, la sensation de chaleur cutanée, les érythèmes, en particulier dus aux ultraviolets, le prurit, l'érythème noueux, l'urticaire, les piqûres d'insectes, les allergies, l'alopécie dans ces phases inflammatoires, les affections articulaires telles que la polyarthrite rhumatoïde, l'arthrose, la tendinite, la périarthrite, les spondylarthropathies ou les atteintes articulaires des entérophaties chroniques, les affections rhumatismales telles que le rhumatisme articulaire aigu, la polyarthrite rhumatoïde, les affections pulmonaires telles que l'emphysème, la mastocytose systémique, le psoriasis, ou encore d'autres affections dermatologiques comme la polychondrite atrophiante, l'érythèmalgie, la nécrobiose lipoïdique. On peut encore citer le lupus érythémateux disséminé.

Quel que soit le phénomène envisagé, il existe un point commun à tous ces mécanismes qui se traduit par une réaction inflammatoire dont la facette terminale peut se mesurer par la libération par les cellules mastocytaires, endothéliales, kératinocytaires, fibroblastiques, mélanocytaires et/ou de Langerhans de la peau d'au moins un médiateur de l'inflammation tel que l'histamine, la sérotonine, l'héparine, les leukotriènes, les prostaglandines, les cytokines, le monoxyde d'azote ou des espèces oxygénées réactives.

Particulièrement, on sait qu'au niveau des couches superficielles de la peau, les kératinocytes en réponse à un signal proinflammatoire (chimiokines, cytokines comme l'interleukine-l) libèrent de l'interleukine-8 ce qui contribue au déclenchement du processus inflammatoire.

On recherche depuis de nombreuses années, dans l'industrie pharmaceutique, des substances permettant de traiter l'inflammation. A cet égard, nombreuses sont celles qui ont déjà été décrites, connue dans la littérature sous les appellations d'anti-inflammatoires stéroïdiens ou non-stéroïdiens (AIS ou AINS) et dont on trouvera une description dans, par exemple, l'ouvrage de Schorderet et Dayer "Pharmacologie, Des concepts fondamentaux aux applications thérapeutiques", 1992, chapitre 37, pages 541-561, 2ième édition, Frison-Roche/Slatkine éditeurs.

Outre que les anti-inflammatoires connus présentent souvent des effets secondaires non négligeables, il demeure intéressant de disposer de nouveaux produits à activité anti-inflammatoire, notamment pour des affections cutanées mineures, telles que, par exemple, les peaux sensibles, l'inconfort cutané, les tiraillements cutanés, les démangeaisons cutanés, le gonflement cutané, la douleur cutanée, la rougeur cutanée, la sensation de chaleur cutanée, les érythèmes, en particulier dus aux ultraviolets, et le prurit.

Le but de la présente invention est donc de pouvoir disposer d'un produit nouveau présentant une activité anti-inflammatoire et pouvant ne pas présenter d'effets secondaires notables.

Ce but et d'autres sont atteints par la présente invention qui a pour objet une composition comprenant à titre de principe actif au moins l'association en une quantité efficace d'au moins un dérivé de l'hormone stimulatrice des mélanocytes de type α (α-MSH) ou de tout équivalent biologique fonctionnel, et d'au moins un extrait d'algue d'origine marine ledit extrait étant une solution d'oligosaccharides obtenue par dépolymérisation enzymatique de polysaccharides membranaires d'algue brune.

Selon l'invention, le dérivé peptidique est un dérivé de l'hormone stimulatrice des mélanocytes de type α (α-MSH) ou Mélanotropine.
L'α-MSH fut décrite à l'origine comme produite par la glande pituitaire, mais le cerveau en général, le sang, la peau et d'autres tissus sont capables également de produire de l'α-MSH.
Ainsi, dans l'épiderme, il a été montré par Schauer et coll. (J. Clin. Invest. 93, May 1994 pp. 2258-2262) que les kératinocytes sont une source d'α-MSH.
Des récepteurs à l'α-MSH sont présents dans de nombreux types cellulaires et notamment dans les follicules pileux de scalp humain (Pigment cell Res. 4:193-8, 1991).

L'αMSH (1-13) est connue pour son activité antipyrétique, son activité anti-inflammatoire et son activité propigmentante. Ce neuropeptide est connu pour inhiber l'inflammation induite par des cytokines ou d'autres médiateurs de l'inflammation ainsi que par des irritants.
Le signal antipyrétique de l'α-MSH réside dans sa séquence carboxy-terminale et peut-être mimé par le tripeptide 11-13 carboxy-terminal (L)Lys(L)Pro(L)Val (Watanabe et al. Brain research Bulletin, Vol. 32, pp. 311-314, 1993).
Ainsi, les brevets US 5028592 et WO 88/00833 visent à protéger l'utilisation du tripeptide (L ou D)Lys-(L)Pro-(L ou D)Val dans un procédé de traitement thérapeutique anti-inflammatoire et dans la préparation d'un médicament pour traiter l'inflammation.

D'autres dérivés de l'α-MSH sont connus pour leur activité anti-inflammatoire. Par exemple la demande de brevet WO 95/08564 décrit l'activité anti-inflammatoire de composés comprenant au moins une séquence de 4 acides aminés de l'α-MSH conjugués avec de l'acide thioctique. Cette demande de brevet est incorporé par référence.

Ainsi, dans la demande de brevet WO 95/08564, on peut citer plus particulièrement les composés I à VII suivants :
I [(DL) Lip] Glu --- His --- D.homoPhe --- Arg --- Trp --- Gly --- NH2
II [(DH Lip] Glu --- His --- D.homoPhe --- Arg --- Trp --- Gly --- NH2
III [(DL) Lip] Glu --- His --- ParafluoroPhe --- Arg --- Trp --- Gly --- NH2
IV [(DL) Lip] His --- D.homoPhe --- Arg --- Trp --- NH2
V [N.Lipoyl-Lysine] Glu --- His -- D.homoPhe -- Arg -- Trp --- Gly -- NH2
VI [N.lipoyl-Lysine] His --- D.homoPhe -- Arg -- Trp -- Gly -- NH2
VII [N.lipoyl-Lysine] His --- D.homoPhe --- Arg --- Trp --- NH2
ainsi que les dérivés de ces molécules sous forme de sels d'esters ou d'amides.

La société SEPORGA commercialise ainsi un produit sous la marque MODULENE® constitué d'un dérivé peptidique d'α-MSH et doué de propriétés anti-inflammatoires.

La demanderesse a de manière surprenante et inattendue découvert que les propriétés anti-inflammatoires de dérivés de l'α-MSH peuvent être améliorées par l'association de ces derniers avec un extrait d'algue d'origine marine ledit extrait étant une solution d'oligosaccharides obtenue par dépolymérisation enzymatique de polysaccharides membranaires d'algue brune.

Ainsi la demanderesse a pu montrer que l'association d'un dérivé peptidique de l'α-MSH et d'un extrait d'algue d'origine marine selon l'invention présente un effet anti-inflammatoire supérieur à la simple addition des effets anti-inflammatoires que peuvent présenter les produits pris isolément. De plus la demanderesse a montré que l'association produit un effet anti-inflammatoire lorsque chacun des produits de l'association est utilisé dans celle-ci a une concentration pour laquelle, utilisés seuls, ils ne produisent aucun effet.

Ainsi, outre l'avantage que l'association présente un effet anti-inflammatoire supérieur à celui des produits pris isolément, elle permet l'utilisation de chacun des produits de l'association à des concentrations inférieures à celles utilisées pour chacun des produits pris isolément.

A cet égard, les exemples présentés ci-après illustrent ces propriétés.

Ainsi, l'invention a pour objet premier une composition comprenant à titre de principe actif au moins l'association en une quantité efficace d'au moins un dérivé peptidique de l'α-MSH ou de tout équivalent biologique fonctionnel, et d'au moins un extrait d'algue d'origine marine ledit extrait étant une solution d'oligosaccharides obtenue par dépolymérisation enzymatique de polysaccharides membranaires d'algue brune.

Par équivalent biologique fonctionnel, on entend un peptide fonctionnellement équivalent en terme de fonction biologique dont l'un au moins des résidus d'acide aminé peut avoir été changé pour un résidu d'acide aminé ayant un index hydropathique similaire.
L'index hydropathique est un index attribué aux acides aminés en fonction de leur hydrophobicité et de leur charge (Kyte et al. (1982), J. Mol. Biol., 157 : 105).

Dans le domaine des acides aminés, la géométrie des molécules est telle qu'elles peuvent théoriquement se présenter sous la forme d'isomères optiques différents. Il existe en effet une conformation moléculaire de l'acide aminé (aa) telle qu'elle dévie à droite le plan de polarisation de la lumière (conformation dextrogyre ou D-aa), et une conformation moléculaire de l'acide aminé (aa) telle qu'elle dévie à gauche le plan de polarisation de la lumière (conformation lévogyre ou L-aa).

La nature n'a retenu pour les acides aminés naturels que la conformation lévogyre. En conséquence si le peptide utilisé dans les compositions selon l'invention est d'origine naturelle, celui-ci sera constitué d'acides aminés de type L-aa.
Cependant, la synthèse chimique en laboratoire permet de préparer des d'acides aminés ayant les deux conformations possibles. A partir de ce matériel de base il est possible d'incorporer lors de la synthèse de peptides aussi bien des d'acides aminés sous la forme d'isomères optiques dextrogyre ou lévogyre.
On peut ainsi incorporer lors de la synthèse de peptides des résidus d'acides aminés Lysine-Proline-Valine indifféremment sous leur forme D-Lysine (D-Lys), L-Lysine (L-Lys), D-Proline (D-Pro), L-Proline (L-Pro), D-Valine (D-Val) ou L-Valine (L-Val).

Ainsi, le dérivé peptidique de l'invention peut être un peptide dont les résidus acides aminés sont indifféremment sous la forme d'isomères optiques dextrogyre ou lévogyre.

On peut citer ainsi les peptides contenant au moins l'un des tripeptides suivant :
D-Lys-D-Pro-D-Val,
D-Lys-D-Pro- L-Val,
D-Lys-L-Pro -D-Val,
L-Lys-D-Pro-D-Val,
D-Lys-L-Pro-L-Val,
L-Lys-D-Pro-L-Val,
L-Lys-L-Pro-D-Val,
L-Lys-L-Pro-L-Val.

Selon l'invention, il peut bien entendu être utilisé plus d'un peptide. Dans ce cas, le mélange de peptides peut être constitué par l'une des combinaisons possibles des peptides ci-dessus décrits.

Il se peut que pour des questions de résistance à la dégradation il soit nécessaire d'utiliser selon l'invention une forme protégée du peptide. La forme de la protection doit évidement être une forme biologiquement compatible. De nombreuses formes de protections biologiquement compatibles peuvent être envisagées comme par exemple l'acylation ou l'acétylation de l'extrémité amino-terminale et/ou l'amidation de l'extrémité carboxy-terminale.

Ainsi, l'invention le peptide de l'invention peut être un peptide sous une forme protégée ou non.

De préférence, on utilise selon l'invention une protection basée sur l'acylation ou l'acétylation de l'extrémité amino-terminale et/ou sur l'amidation de l'extrémité carboxy-terminale.

Particulièrement, selon l'invention le dérivé peptidique de l'α-MSH est choisi parmi les dérivés peptidiques comprenant au moins le tripeptide Lys-Pro-Val, les dérivés peptidiques comprenant au moins une séquence de 4 acides aminés de l'α-MSH conjugués ou non avec de l'acide thioctique et plus précisément les composés décrits dans la demande de brevet WO 95/08564.

De préférence, on utilise les composés I à VII suivants :
I [(DL) Lip] Glu --- His - D.homoPhe --- Arg --- Trp --- Gly --- NH2
II [(DH Lip] Glu --- His --- D.homoPhe --- Arg --- Trp --- Gly --- NH2
III [(DL) Lip] Glu --- His --- ParaFluoroPhe --- Arg --- Trp --- Gly --- NH2
IV [(DL) Lip] His --- D.homoPhe --- Arg --- Trp --- NH2
V [N.Lipoyl-Lysine] Glu --- His -- D.homoPhe -- Arg -- Trp --- Gly -- NH2
VI [N.lipoyl-Lysine] His -- D.homoPhe -- Arg -- Trp -- Gly -- NH2
VII [N.lipoyl-Lysine] His --- D.homoPhe --- Arg --- Trp --- NH2
ainsi que les dérivés de ces molécules sous forme de sels d'esters ou d'amides.

Un dérivé peptidique comprenant au moins le tripeptide Lys-Pro-Val préférentiellement utilisé selon l'invention est le tripeptide Lys-Pro-Val lui-même et plus particulièrement le tripeptide Lys-Pro-Val pour lequel au moins le résidu d'acide aminé Proline est dans la conformation non naturelle dextrogyre (résidu DPro).

Un autre dérivé préférentiellement utilisé selon l'invention est le dérivé vendu sous la dénomination MODULENE® par la société SEPORGA.

Le peptide utilisé selon l'invention peut bien entendu être d'origine naturelle. Cela sous-entend qu'il peut avoir été purifié à partir de matériel biologique naturel. On peut à cet égard citer en exemple l'α-MSH, largement présente dans le système nerveux central et qu'il est entre autre possible de purifier à partir de glande pituitaire.
Cependant, avec les progrès du génie chimique, il est maintenant aisé de synthétiser à façon des peptides, même de longueur importante.

Ainsi, le dérivé peptidique de l'invention peut être un peptide d'origine naturelle ou synthétique.

Dans la composition de l'invention, le dérivé peptidique peut-être un mélange de dérivés peptidiques.

L'extrait d'algue d'origine marine est une solution d'oligosaccharides obtenue par dépolymérisation enzy matique de polysaccharides membranaires d'algue brune.

Préférentiellement, ledit extrait d'algues d'origine marine est un extrait d'algues brunes de la famille des Laminaires. Encore plus préférentiellement, l'algue brune est une algue de l'espèce *Laminaria digitata.*

Un extrait particulièrement préféré est une solution d'oligosaccharides obtenue par dépolymérisation enzymatique de polysaccharides membranaires d'algue brune, tel que notamment décrit dans la demande de brevet FR2753628, incorporée ici par référence.

A cet égard, un extrait d'algue d'origine marine particulièrement préféré selon l'invention est un extrait vendu par la société CODIF INTERNATIONAL, sous la dénomination PHYCOSACCHARIDES ANTI-INFLAMMATION® qui est une solution concentrée d'un oligosaccharide obtenu par dépolymérisation enzymatique contrôlée de polysaccharides membranaires d'une algue brune. Il comprend l'enchaînement de deux acides uriques : acide mannuronique et l'acide guluronique.

Bien entendu, la composition de l'invention est une composition destinée à un usage cosmétique ou pharmaceutique.

La quantité de chacun des éléments de l'association utilisable selon l'invention dépend bien évidemment de l'effet recherché et doit être en une quantité efficace pour que l'association présente l'effet recherché, particulièrement un effet anti-inflammatoire.

Ainsi pour donner un ordre de grandeur, la composition de l'invention peut comprendre le dérivé peptidique en une quantité pondérale représentant de 10⁻⁶ % à 10 % du poids total de la composition et préférentiellement en une quantité représentant de 10⁻³ % à 5 % du poids total de la composition.

De même pour donner un ordre de grandeur, la composition de l'invention peut comprendre l'extrait d'algue en une quantité pondérale représentant de 0,01 % à 10 % du poids total de la composition et préférentiellement en une quantité représentant de 0,02 % à 5 % du poids total de la composition.

L'invention a également pour objet l'utilisation, à titre de principe actif, de l'association en une quantité efficace d'au moins un dérivé peptidique de l'α-MSH ou de tout équivalent biologique fonctionnel, et d'au moins un extrait d'algue d'origine marine dans une composition ou pour la préparation d'une composition, l'association ou la composition étant destinées à traiter l'inflammation ledit extrait étant une solution d'oligosaccharides obtenue par dépolymérisation enzymatique de polysaccharides membranaires d'algue brune.

Selon cet aspect particulier de l'invention, l'association d'un dérivé peptidique de l'α-MSH ou de tout équivalent biologique fonctionnel, et d'un extrait d'algue d'origine marine est telle que définie précédemment dans le texte.

L'invention a outre pour objet l'utilisation, à titre de principe actif, de l'association en une quantité efficace d'au moins un dérivé peptidique de l'α-MSH ou de tout équivalent biologique fonctionnel, et d'au moins un extrait d'algue d'origine marine ledit extrait étant une solution d'oligosaccharides obtenue par dépolymérisation enzymatique de polysaccharides membranaires d'algue brune dans une composition ou pour la préparation d'une composition, l'association ou la composition étant destinée à inhiber partiellement ou totalement la production d'interleukine-8, particulièrement par les kératinocytes de la peau.

On a vu préalablement dans le texte des exemples de désordres faisant intervenir un processus inflammatoire.

Ces désordres inflammatoires peuvent être cutanés ou systémiques.

Ainsi, les compositions utilisant l'association d'au moins un dérivé peptidique de l'α-MSH et d'au moins un extrait d'algue d'origine marine selon l'invention sont destinées à combattre les désordres faisant intervenir un processus inflammatoire et plus particulièrement les désordres cutanés.

Particulièrement les compositions selon l'invention sont destinées à combattre les affections cutanées telles que les peaux sensibles, l'inconfort cutané, les tiraillements cutanés, les démangeaisons cutanés, le gonflement cutané, la douleur cutanée, la rougeur cutanée, la sensation de chaleur cutanée, les érythèmes, en particulier dus aux ultraviolets, le prurit, l'érythème noueux, l'urticaire, les piqûres d'insectes, les allergies, l'alopécie dans ces phases inflammatoires.

Encore plus particulièrement les compositions selon l'invention sont destinées à combattre les irritations cutanées et/ou les dartres et/ou les sensations dysesthésiques et/ou les sensations d'échauffement et/ou les prurits de la peau et/ou des muqueuses.

Quelque soit la forme de l'invention, la composition selon l'invention peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.
Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits après-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

La composition selon l'invention peut également consister en des préparations solides constituant des savons ou des pains de nettoyage.

La composition peut aussi être conditionnée sous forme d'une composition pour aérosol comprenant également un agent propulseur sous pression.

La composition selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une lotion ou un gel antichute, un shampooing antiparasitaire, etc.

La composition peut aussi être à usage bucco-dentaire, par exemple une pâte dentifrice. Dans ce cas, la composition peut contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose R 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

La présente invention a en outre pour objet une composition cosmétique, caractérisé par le fait que ladite composition est destinée à l'application sur la peau, sur les cheveux, et/ou sur les muqueuses, une composition telle que décrite ci-dessus.

La présente invention a plus particulièrement pour objet une composition cosmétique ayant un effet apaisant cutané, caractérisé par le fait que ladite composition est destinée à l'application sur la peau, sur les cheveux, et/ou sur les muqueuses, une composition telle que décrite ci-dessus.

La composition cosmétique de l'invention peut être utilisée notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires ou après-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions les proportions indiquées sont des pourcentages en poids, sauf mention contraire.

### Exemple 1 : dosage de l'interleukine-8 induite par l'interleukine-1 dans le surnageant de cellules DK7

### Principe et but de l'étude :

Ce test permet d'évaluer le potentiel anti-inflammatoire de molécules diverses, sur une lignée cellulaire kératinocytaire (DK7). Dans ce test on mime une situation inflammatoire en exacerbant la production d'IL-8 des DK7 par l'ajout d'IL-1α dans le milieu de culture. On mesure, ensuite, l'effet d'une molécule par son action inhibitrice vis à vis de cette production exacerbée.

Origine des cellules : cellules humaines immortalisées (infectées SV40-T-Ag + Human Papilloma Virus 16 E6/E7) non tumorales appelées DK7-Nestlé Recherchedécrites dans la demande de brevet PCT/EP96/05812 (Société des produits Nestlé).

### Mode opératoire :

Des cellules DK7 conservées sous forme congelées sont préalablement mises en culture selon les protocoles classiques dans un flask de 75cm² préalablement recouvert par une solution de « coating » (5 mg de fibronectine humaine (Sigma) + 5 ml de vitrogen 100 (collagène bovin purifié pour culture/PC0701/ Collagen corporation) + 50 ml d'une solution à 0.1% de BSA (BSA/ réf:343020/Biofluids) + 440 ml de milieu NR1), en présence de 20 ml de milieu NR2 (A partir de 500ml de milieu sérum free de base NR1 (biofluids n° P185-500), ajouter 2.5 ml d'extrait pituitaire bovin (BPE) (biofluids n° 210) et 5 ml de antibiotic/antimycotic (réf : 15240-C39/GIBCO)). Les cellules sont alors cultivées jusqu'à la confluence.

Les cellules sont alors détachées du flask par trypsinisation selon les techniques classiques. A J=0, les cellules sont ensemencées sur une(des) plaque(s) pré-coatée de 96 puits à raison de 200µl de milieu par puits (densité cellulaire : 6.10⁴ cellules /ml).
A J=1, les cellules sont mises en contact avec le produit à tester.
30 à 50 minutes après le traitement de l'IL-1 à 2,5ng/ml est ajoutée au milieu de culture.

Les cellules sont alors incubées pendant 24 heures à 37°C .
Le dosage d'IL-8 dans les surnageants ainsi qu'un dosage de protéines précédé par un test XTT/BrdU sont alors effectués.

### Dosage IL-8 :

Ce dosage est réalisé à l'aide d'un kit ELISA/IL-8 (code RPN 2764/Biotrak/Amersham) selon les données du fournisseur.
Le dosage s'effectue sur un volume de 50µl de milieu de culture.
La densité optique est lue à 450 nm à l'aide d'un spectrophotomètre «Labsystems Multisckan Multisoft ».

### Dosage de protéine :

Un dosage de protéines est effectué sur chaque échantillon à l'aide d'un kit dosage protéine (BCA protein assay kit/ réf: 23225/Pierce) conservé à température ambiante.

### Produits testés :

DM1 = MODULENE® à 1 µM ;
DM10 = MODULENE® à 10 µM ;
Phyco = PHYCOSACCHARIDES ANTI-INFLAMMATION® à 50 µM
DM1+ Phyco = MODULENE® à 1 µM = PHYCOSACCHARIDES ANTI-INFLAMMATION® à 50 µM
DM10+ Phyco = MODULENE® à 10 µM = PHYCOSACCHARIDES ANTI-INFLAMMATION® à 50 µM
Contrôle : Cellules traitées à l'IL-1 et non traitée avec l'un des composés à tester.

### Résultats :

Les données du dosage IL-8 sont exprimées en pg d'IL-8 par µg de protéines:

| | IL-8 | % inhibition |
|---|---|---|
| Contrôle | 44,04 | - |
| DM1+ Phyco | 24,73 | 43,8 |
| DM10 + Phyco | 22,71 | 48,4 |
| DM1 | 43,69 | 0,8 |
| DM10 | 34,89 | 20,8 |
| Phyco | 52,95 | 0 |

L'association DM + phyco inhibe de façon significative la production d'IL-8 produite dans le lot contrôle.
L'association DM1 + Phyco inhibe la production d'IL-8, alors que DM1 seul n'a aucun effet sur IL-8. Ce résultat démontre l'effet de synergie de l'association MODULENE® + PHYCOSACCHARIDES ANTI-INFLAMMATION®.

### Exemple 2 : Exemples de formulations illustrant l'invention. Ces compositions ont été obtenues par simple mélange des différents composants.

| Composition 1 : Crème de jour : | |
|---|---|
| Phycosaccharide anti-inflammation®* | 5,00% |
| Modulene®** | 1,00% |
| stéarate de sucrose | 4,00% |
| alcool stéarylique | 2,00% |
| cyclohexasiloxane | 9,00% |
| huile minérale | 4,00% |
| glycérine | 5,00% |
| gomme de xanthane | 0.30% |
| carbomer | 0.50% |
| conservateurs | 0.30% |
| parfum | 0.30% |
| eau | qsp100 |

| Composition 2 : fluide de soin : | |
|---|---|
| Phycosaccharide anti-inflammation®* | 1,00% |
| Modulene®** | 1,00% |
| alcool stéarylique | 0.40% |
| stéarate de sorbitan | 1.50% |
| glycérine | 5,00% |
| gomme de xanthane | 0.20% |
| carbomer | 0.10% |
| cyclohexasiloxane | 7,00% |
| conservateurs | 0.30% |
| parfum | 0.20% |
| eau | qsp 100 |

| Composition 3 : lotion : | |
|---|---|
| Phycosaccharide anti-inflammation®* | 0.02% |
| Modulene®** | 1,00% |
| propylenglycol | 2,00% |
| extrait de fleurs de bleuet | 0.10% |
| conservateurs | 0.10% |
| PEG 60 hydrogenated castor oil | 0.40% |
| parfum | 0.10% |
| eau | qsp100 |

| | |
|---|---|
| Phycosaccharide anti-inflammation®* : oligosaccharide origine Laminaria digitata (P.M. 3500 daltons) à 5% dans de l'eau | |
| Modulene®** : lipoylpeptide stabilisé par dextrane (1%) en solution aqueuse stabilisée (Phenonip 0,3%) | |

## Revendications

1. Composition comprenant à titre de principe actif au moins l'association en une quantité efficace d'au moins un dérivé peptidique de l'α-MSH (hormone stimulatrice des mélanocytes de type α) ou de tout équivalent biologique fonctionnel, et d'au moins un extrait d'algue d'origine marine, ledit extrait étant une solution d'oligosaccharides obtenue par dépolymérisation enzymatique de polysaccharides membranaires d'algue brune.

2. Composition selon la revendication précédente, **caractérisée par le fait que** dérivé peptidique de l'α-MSH est choisi parmi les dérivés peptidiques comprenant au moins le tripeptide Lys-Pro-Val, les dérivés peptidiques comprenant au moins une séquence de 4 acides aminés de l'α-MSH conjugués avec de l'acide thioctique.

3. Composition selon la revendication précédente, **caractérisée par le fait que** dérivé peptidique comprenant au moins le tripeptide Lys-Pro-Val est le tripeptide Lys-Pro-Val.

4. Composition selon la revendication précédente, **caractérisée par le fait que** dérivé peptidique comprenant au moins le tripeptide Lys-Pro-Val est le tripeptide Lys-Pro-Val pour lequel au moins le résidu d'acide aminé Pro est dans la conformation non naturelle dextrogyre.

5. Composition selon la revendication 2, **caractérisée par le fait que** dérivé peptidique de l'α-MSH est choisi parmi les composés I à VII suivants :
I [(DL) Lip] Glu --- His --- D.homoPhe --- Arg --- Trp --- Gly --- NH2
II [(DH Lip] Glu --- His --- D.homoPhe --- Arg --- Trp *---* Gly --- NH2
III [(DL) Lip] Glu --- His --- ParaFluoroPhe -- Arg --- Trp --- Gly --- NH2
IV [(DL) Lip] His --- D.homoPhe --- Arg --- Trp --- NH2
V [N.Lipoyl-Lysine] Glu --- His --- D.homoPhe -- Arg -- Trp --- Gly -- NH2
VI [N.lipoyl-Lysine] His --- D.homoPhe -- Arg -- Trp -- Gly -- NH2
VII [N.lipoyl-Lysine] His -- D.homoPhe -- Arg ... Trp ... NH2
VII [N.lipoyl-Lysine] His - D.homoPhe - Arg ... Trp ... NH2
ainsi que les dérivés de ces molécules sous forme de sels d'esters ou d'amides.

6. Composition selon l'une quelconque des revendications 1 à 5, **caractérisée par le fait que** l'extrait d'algue d'origine marine est un extrait d'algues brunes de la famille des Laminaires.

7. Composition selon la revendication précédente, **caractérisée par le fait que** l'algue brune est de l'espèce *Laminaria digitata.*

8. Composition selon l'une quelconque des revendications précédentes, **caractérisée par le fait que** le dérivé peptidique est en une quantité pondérale représentant de 10⁻⁶ % à 10 % du poids total de la composition.

9. Composition selon la revendication précédente, **caractérisée par le fait que** le dérivé peptidique est en une quantité représentant de 10⁻³ % à 5 % du poids total de la composition.

10. Composition selon l'une quelconque des revendications précédentes, **caractérisée par le fait que** l'extrait d'algue est en une quantité pondérale représentant de 0,01 % à 10 % du poids total de la composition

11. Composition selon la revendication précédente, **caractérisée par le fait que** l'extrait d'algue est en une quantité représentant de 0,02 % à 5 % du poids total de la composition.

12. Utilisation, à titre de principe actif, de l'association en une quantité efficace d'au moins un dérivé peptidique de l'α-MSH ou de tout équivalent biologique fonctionnel, et d'au moins un extrait d'algue d'origine marine constitué d'une solution d'oligosaccharides obtenue par dépolymérisation enzymatique de polysaccharides membranaires d'algue brune, pour la préparation d'une composition, l'association ou la composition étant destinées à traiter l'inflammation.

13. Utilisation, à titre de principe actif, de l'association en une quantité efficace d'au moins un dérivé peptidique de l'α-MSH ou de tout équivalent biologique fonctionnel, et d'au moins un extrait d'algue d'origine marine constitué d'une solution d'oligosaccharides obtenue par dépolymérisation enzymatique de polysaccharides membranaires d'algue brune, pour la préparation d'une composition, l'association ou la composition étant destinée à inhiber partiellement ou totalement la production d'interleukine-8, particulièrement par les kératinocytes de la peau.

14. Utilisation, à titre de principe actif, de l'association en une quantité efficace d'au moins un dérivé peptidique de l'α-MSH ou de tout équivalent biologique fonctionnel, et d'au moins un extrait d'algue d'origine marine constitué d'une solution d'oligosaccharides obtenue par dépolymérisation enzymatique de polysaccharides membranaires d'algue brune, pour la préparation d'une composition, l'association ou la composition étant destinée à combattre les désordres cutanés.

15. Utilisation selon la revendication précédente, **caractérisée en ce que** les désordres cutanés sont choisis parmi : les peaux sensibles, l'inconfort cutané, les tiraillements cutanés, les démangeaisons cutanés, le gonflement cutané, la douleur cutanée, la rougeur cutanée, la sensation de chaleur cutanée, les érythèmes, en particulier dus aux ultraviolets, le prurit, l'érythème noueux, l'urticaire, les piqûres d'insectes, les allergies, l'alopécie dans ces phases inflammatoires.

16. Utilisation selon l'une quelconque des revendications 14 et 15, **caractérisée en ce que** les désordres cutanés sont choisis parmi : les irritations cutanées et/ou les dartres et/ou les sensations dysesthésiques et/ou les sensations d'échauffement et/ou les prurits de la peau et/ou des muqueuses.

17. Utilisation d'au moins dérivé peptidique de l'α-MSH ou de tout équivalent biologique fonctionnel, et d'au moins un extrait d'algue d'origine marine constitué d'une solution d'oligosaccharides obtenue par dépolymérisation enzymatique de polysaccharides membranaires d'algue brune, pour la préparation d'une composition selon l'une des revendications 1 à 11 , **caractérisé en ce que** ladite composition est une composition cosmétique pour application sur la peau, sur les cheveux, et/ou sur les muqueuses.

18. Utilisation d'au moins dérivé peptidique de l'α-MSH ou de tout équivalent biologique fonctionnel, et d'au moins un extrait d'algue d'origine marine constitué d'une solution d'oligosaccharides obtenue par dépolymérisation enzymatique de polysaccharides membranaires d'algue brune, pour la préparation d'une composition selon la revendication précédente, **caractérisé en ce que** ladite composition est une composition cosmétique ayant un effet apaisant cutané.

## Claims

1. Composition which comprises, as the active principle, at least the combination of an effective quantity of at least one peptide derivative of α-MSH (α-type melanocyte stimulating hormone), or any functional biological equivalent, and at least one algal extract of marine origin, said extract being an oligosaccharide solution which is obtained by the enzymic depolymerization of brown algal membrane polysaccharides.

2. Composition according to the preceding claim, **characterized in that** the peptide derivative of α-MSH is selected from the peptide derivatives comprising at least the tripeptide Lys-Pro-Val, and the peptide derivatives comprising at least one 4-amino acid sequence from α-MSH conjugated to thioctic acid.

3. Composition according to the preceding claim, **characterized in that** the peptide derivative comprising at least the tripeptide Lys-Pro-Val is the tripeptide Lys-Pro-Val.

4. Composition according to the preceding claim, **characterized in that** the peptide derivative comprising at least the tripeptide Lys-Pro-Val is the tripeptide Lys-Pro-Val in which at least the amino acid residue Pro is in the unnatural dextrorotatory conformation.

5. Composition according to Claim 2, **characterized in that** the peptide derivative of α-MSH is selected from the following compounds I to VII:
I [(DL)Lip] Glu---His---D.homoPhe---Arg---Trp---Gly---NH2
II [(DH Lip] Glu---His---D.homoPhe---Arg---Trp---Gly---NH2
III [(DL)Lip] Glu---His---ParaFluoroPhe---Arg---Trp---Gly---NH2
IV [(DL)Lip] His---D.homoPhe---Arg---Trp---NH2
V [N.lipoyl-Lysine] Glu---His---D.homoPhe---Arg---Trp---Gly---NH2
VI [N.lipoyl-Lysine] His---D.homoPhe---Arg---Trp---Gly---NH2
VII [N.lipoyl-Lysine] His---D.homoPhe---Arg---Trp---NH2
as well as the derivatives of these molecules in the form of salts of esters or amides.

6. Composition according to any one of Claims 1 to 5, **characterized in that** the algal extract of marine origin is an extract of brown algae of the Laminaria family.

7. Composition according to the preceding claim, **characterized in that** the brown alga is of the species *Laminaria digitata.*

8. Composition according to any one of the preceding claims, **characterized in that** the peptide derivative is in a quantity by weight which represents from 10⁻⁶% to 10% of the total weight of the composition.

9. Composition according to the preceding claim, **characterized in that** the peptide derivative is in a quantity which represents from 10⁻³% to 5% of the total weight of the composition.

10. Composition according to any one of the preceding claims, **characterized in that** the algal extract is in a quantity by weight which represents from 0.01% to 10% of the total weight of the composition.

11. Composition according to the preceding claim, **characterized in that** the algal extract is in a quantity which represents from 0.02% to 5% of the total weight of the composition.

12. Use, as the active principle, of the combination of an effective quantity of at least one peptide derivative of α-MSH, or any functional biological equivalent, and at least one algal extract of marine origin constituted of an oligosaccharide solution which is obtained by the enzymic depolymerization of brown algal membrane polysaccharides, for the preparation of a composition, with the combination or the composition being intended for treating inflammation.

13. Use, as the active principle, of the combination of an effective quantity of at least one peptide derivative of α-MSH, or any functional biological equivalent, and at least one algal extract of marine origin constituted of an oligosaccharide solution which is obtained by the enzymic depolymerization of brown algal membrane polysaccharides, for the preparation of a composition, with the combination or the composition being intended for partially or totally inhibiting the production of interleukin 8, in particular by the keratinocytes of the skin.

14. Use, as the active principle, of the combination of an effective quantity of at least one peptide derivative of α-MSH, or any functional biological equivalent, and at least one algal extract of marine origin constituted of an oligosaccharide solution which is obtained by the enzymic depolymerization of brown algal membrane polysaccharides, for the preparation of a composition, with the combination or the composition being intended for controlling skin disorders.

15. Use according to the preceding claim, **characterized in that** the skin disorders are selected from: sensitive skins, skin discomfort, skin stretching, skin itching, skin swelling, skin pain, skin flushing, heat sensation of the skin, erythemas, in particular due to ultraviolet rays, pruritus, erythema nodosum, urticaria, insect bites, allergies and alopecia in its inflammatory phases.

16. Use according to either one of Claims 14 and 15, **characterized in that** the skin disorders are selected from: skin irritations and/or sores and/or dysaesthesic sensations and/or heating sensations and/or prurituses of the skin and/or the mucous membranes.

17. Use of at least one peptide derivative of α-MSH, or any functional biological equivalent, and at least one algal extract of marine origin constituted of an oligosaccharide solution which is obtained by the enzymic depolymerization of brown algal membrane polysaccharides, for the preparation of a composition according to one of Claims 1 to 11, **characterized in that** the said composition is a cosmetic composition to be applied to the skin, to the hair and/or to the mucous membranes.

18. Use of at least one peptide derivative of α-MSH, or any functional biological equivalent, and at least one algal extract of marine origin constituted of an oligosaccharide solution which is obtained by the enzymic depolymerization of brown algal membrane polysaccharides, for the preparation of a composition according to the preceding claim, **characterized in that** the said composition is a cosmetic composition having a skin-soothing effect.

## Patentansprüche

1. Zusammensetzung, die als Wirkstoff mindestens eine Kombination von mindestens einem Peptidderivat von α-MSH (Melanozyten-stirnuliexendes Hormon Typ α) oder einem biologischen funktionellen Äquivalent mit mindestens einem Extrakt von Algen marinen Ursprungs in einer wirksamen Menge enthält, wobei der Extrakt eine Lösung von Oligosacchariden ist, die durch enzymatische Depolymerisation von Membran-Polysacchariden von Braunalgen erhalten ist.

2. Zusammensetzung nach dem vorhergehenden Anspruch, **dadurch gekennzeichnet, dass** das Peptidderivat von α-MSH ausgewählt ist unter Peptidderivaten, die mindestens das Tripeptid Lys-Pro-Val enthalten, wobei die Peptidderivate mindestens eine Sequenz von 4 Aminosäuren von α-MSH enthalten, die mit Thioct-Säure konjugiert sind.

3. Zusammenfassung nach dem vorhergehenden Anspruch, **dadurch gekennzeichnet, dass** das Peptidderivat, das mindestens das Tripeptid Lys-Pro-Val enthält, das Tripeptid Lys-Pro-Val ist.

4. Zusammensetzung nach dem vorhergehenden Anspruch, **dadurch gekennzeichnet, dass** das Peptidderivat, das mindestens das Tripeptid Lys-Pro-Val enthält, das Tripeptid Lys-Pro-Val ist, bei dem zumindest der Aminosäurerest der Aminosäure Pro in der nicht natürlichen rechtsdrehenden Konformation vorliegt.

5. Zusammensetzung nach Anspruch 2, **dadurch gekennzeichnet, dass** das Peptidderivat von α-MST unter den folgenden Verbindungen I bis VII ausgewählt ist:
I [(D, L) Lip] Glu - His - D-homoPhe - Arg - Trp - Gly - NH₂
II [(D, H) Lip] Glu - His - D-homoPhe - Arg - Trp - Gly - NH₂
III [(D, L) Lip] Glu - His - p-Fluor-Phe - Arg - Trp - Gly - NH₂
IV [(D, L) Lip] His - D-homoPhe - Arg - Trp - NH₂
V [N-Lipoyl-Lysin] Glu - His - D-homoPhe - Arg - Trp - Gly - NH₂
VI [N-Lipoyl-Lysin] His - D-homoPhe - Arg - Trp - Gly - NH₂
VII [N-Lipoyl-Lysin] His - D-homoPhe - Arg - Trp - NH₂
sowie den Derivaten dieser Moleküle in Form von Salzen, Estern oder Amiden.

6. Zusammensetzung nach einem der Ansprüche 1, bis 5, **dadurch gekennzeichnet, dass** der Extrakt von Algen marinen Ursprungs ein Extrakt von Braunalgen der Gattung der Laminaria ist.

7. Zusammensetzung nach dem vorhergehenden Anspruch, **dadurch gekennzeichnet, dass** die Braunalge von der Art *Laminaria digitata* ist.

8. Zusammensetzung nach einem der vorhergehenden Ansprüche, **dadurch gekennzeichnet, dass** das Peptidderivat in einer Gewichtsmenge vorliegt, die 10⁻⁶ bis 10 % des Gesamtgewichts der Zusammensetzung ausmacht.

9. Zusammensetzung nach dem vorhergehenden Anspruch, **dadurch gekennzeichnet, dass** das Peptidderivat in einer Menge vorliegt, die 10⁻³ bis 5 % des Gesamtgewichts der Zusammensetzung ausmacht.

10. Zusammensetzung nach einem der vorhergehenden Ansprüche, **dadurch gekennzeichnet, dass** der Algenextrakt in einer Gewichtsmenge vorliegt, die 0,01 bis 10 % des Gesamtgewichts der Zusammensetzung ausmacht.

11. Zusammensetzung nach dem vorhergehenden Anspruch, **dadurch gekennzeichnet, dass** der Algenextrakt in einer Menge vorliegt, die 0,02 bis 5 % des Gesamtgewichts der Zusammensetzung ausmacht.

12. Verwendung der Kombination mindestens eines Peptidderivats von α-MSH oder eines biologischen funktionellen Äquivalents mit mindestens einem Extrakt von Algen marinen Ursprungs, der aus einer Lösung von Oligosacchariden besteht, die durch enzymatische Depolymerisation von Membran-Polysacchariden von Braunalgen erhalten ist, als Wirkstoff in einer wirksamen Menge zur Herstellung einer Zusammensetzung, wobei die Kombination oder die Zusammensetzung zur Behandlung von Entzündungen vorgesehen ist.

13. Verwendung der Kombination mindestens eines Peptidderivats von α-MSH oder eines biologischen funktionellen Äquivalents mit mindestens einem Extrakt von Algen marinen Ursprungs, der aus einer Lösung von Oligosacchariden besteht, die durch enzymatische Depolymerisation von Membran-Polysacchariden von Braunalgen erhalten ist, als Wirkstoff in einer wirksamen Menge zur Herstellung einer Zusammensetzung, wobei die Kombination oder die Zusammensetzung dazu vorgesehen ist, die Produktion von Interleukin-8, insbesondere durch die Keratinocyten der Haut, teilweise oder vollständig zu inhibieren.

14. Verwendung der Kombination mindestens eines Peptidderivats von α-MSH oder eines biologischen funktionellen Äquivalents mit mindestens einem Extrakt von Algen marinen Ursprungs, der aus einer Lösung von Oligosacchariden besteht, die durch enzymatische Depolymerisation von Membran-Polysacchariden von Braunalgen erhalten ist, als Wirkstoff in einer wirksamen Menge zur Herstellung einer Zusammensetzung, wobei die Kombination oder die Zusammensetzung zur Bekämpfung von Hautstörungen vorgesehen ist.

15. Verwendung nach dem vorhergehenden Anspruch, **dadurch gekennzeichnet, dass** die Hautstörungen ausgewählt sind unter empfindlicher Haut, unangenehmem Hautgefühl, Ziehen der Haut, Hautjucken, Hautschwellungen, schmerzhafter Haut, Hautrötung, Hitzeempfindung der Haut, Erythemen, insbesondere UV-bedingten Erythemen, Pruritus, knotigem Erythem, Urticaria, Insektenstichen, Allergien und Alopezie in ihren entzündlichen Phasen.

16. Verwendung nach Anspruch 14 oder 15, **dadurch gekennzeichnet, dass** die Hautstörungen ausgewählt sind unter Hautreizungen und/oder Flechten und/oder unangenehmen Hautempfindungen und/oder Hitzempfindungen der Haut und/oder Pruriti der Haut und/oder der Schleimhäute.

17. Verwendung mindestens eines Peptidderivats von α-MSH oder eines biologischen funktionellen Äquivalents und mindestens eines Extrakts von Algen marinen Ursprungs, der aus einer Lösung von Oligosacchariden besteht, die durch enzymatische. Depolymerisation von Membran-Polysacchariden von Braunalgen erhalten ist, zur Herstellung einer Zusammensetzung nach einem der Ansprüche 1 bis 11, **dadurch gekennzeichnet, dass** die Zusammensetzung eine kosmetische Zusammensetzung zum Aufbringen auf die Haut, die Haare und/oder die Schleimhäute ist.

18. Verwendung mindestens eines Peptidderivats von α-MSH oder eines biologischen funktionellen Äquivalents und mindestens eines Extrakts von Algen marinen Ursprungs, der aus einer Lösung von Oligosacchariden besteht, die durch enzymatische Depolymerisation von Membran-Polysacchariden von Braunalgen erhalten ist, zur Herstellung einer Zusammensetzung nach dem vorhergehenden Anspruch, **dadurch gekennzeichnet, dass** die Zusammensetzung eine kosmetische Zusammensetzung mit hautberuhigender Wirkung ist.
